# EUROPÄISCHE PATENTANMELDUNG

(11) **EP 4 803 180 A1**
(43) Veröffentlichungstag der Anmeldung: **09.09.2026**
(21) Anmeldenummer: 25162052.2
(22) Anmeldetag: 06.03.2025
(51) Int. Cl.: B01D 61/44, C02F 1/469, C07H 15/04, C07H 15/06, C11B 1/10

(54) **ELEKTROCHEMISCHES ISOLIEREN VON RHAMNOLIPIDEN AUS WÄSSRIGEN MEDIEN**

(71) Anmelder: Evonik Operations GmbH, 45128 Essen (DE)
(72) Erfinder: KOKOT, Linda, 55252 Mainz-Kastel (DE); MÜLLER, Jakob, 45721 Haltern am See (DE); HELLRIEGEL, Jan, 55120 Mainz (DE); STENNER, Patrik, 63452 Hanau (DE); BLANK-SHIM, Silvia, 63743 Aschaffenburg (DE)
(74) Vertreter: Evonik Patent Association

(57) **Zusammenfassung**

Die Erfindung befasst sich mit der industriellen Aufarbeitung von biotechnologisch hergestellten Rhamnolipiden. Ihr lag die Aufgabe zugrunde, ein Verfahren zur Isolation von Rhamnolipiden aus wässrigen Medien anzugeben, welches die Salzfracht reduziert und möglichst ohne organische Lösemittel auskommt. Zudem soll das Verfahren möglichst energieeffizient sein. Es soll Rhamnolipide mit hoher Reinheit liefern und sich im industriellen Maßstab anwenden lassen. Das erfindungsgemäße Verfahren nutzt das bewährte Absenken und Erhöhen des pH-Wertes, um die Rhamnolipide anhand ihrer Dichte abzuscheiden. Allerdings wird der pH-Wert elektrochemisch eingestellt, nämlich durch Elektrolyse von Wasser. Ein wesentlicher Aspekt des vorliegenden Verfahrens ist demnach, dass sowohl die Absenkung, als auch die Erhöhung des pH-Wertes elektrochemisch durch Wasserspaltung erfolgt. Dies hat den entscheidenden Vorteil, dass keine anorganischen Säuren oder Laugen eingesetzt werden müssen.

## Beschreibung

Die Erfindung befasst sich mit der industriellen Aufarbeitung von biotechnologisch hergestellten Rhamnolipiden.

Rhamnolipide sind eine Klasse von Verbindungen, welche der Formel I gehorchen:

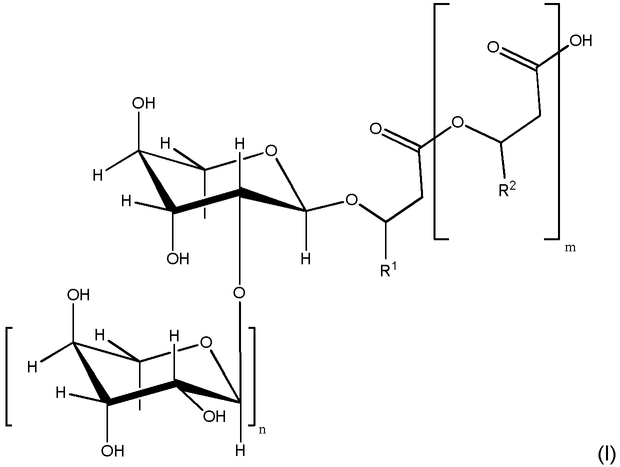

Die in Formel I verwendeten Substituenten sind wie folgt definiert:
m = 2, 1 oder 0, insbesondere 1 oder 0;
n = 1 oder 0, insbesondere 1;
R¹ und R² = unabhängig voneinander gleicher oder verschiedener organischer Rest mit 2 bis 24, bevorzugt 5 bis 13 Kohlenstoffatomen, insbesondere gegebenenfalls verzweigter, gegebenenfalls substituierter, insbesondere hydroxy-substituierter, gegebenenfalls ungesättigter, insbesondere gegebenenfalls einfach, zweifach oder dreifach ungesättigter, Alkylrest, bevorzugt solcher ausgewählt aus der Gruppe bestehend aus Pentenyl, Heptenyl, Nonenyl, Undekenyl und Tridekenyl und (CH₂)o-CH₃ mit o = 1 bis 23, bevorzugt 4 bis 12.

Rhamnolipide im Sinne dieser Erfindung umfassen auch Salze der Verbindungen gemäß Formel (I), insbesondere als Natrium- oder Kaliumsalz, aber auch Magnesium-, Calcium- sowie Aluminium- oder Ammonium-Salze sind möglich.

Rhamnolipide werden als Tensid in Wasch- oder Reinigungsmitteln sowie in kosmetischen Formulierungen eingesetzt. Da Rhamnolipide sich biotechnologisch aus nachwachsenden Rohstoffen erzeugen lassen, werden Rhamnolipide auch als "Biotenside" vermarktet.

### Stand der Technik

Die Herstellung von Rhamnolipiden ist recht gut dokumentiert. So ist das allgemeine Fachwissen unter anderem repräsentiert durch:
Sang-Jin Suh, Krutika Invally, Lu-Kwang Ju, / Chapter 5 - Rhamnolipids: Pathways, Productivities, and Potential. In: Biobased Surfactants (Second Edition), Editor(s): Douglas G. Hayes, Daniel K.Y. Solaiman, Richard D. Ashby, DOI 10.1016/B978-0-12-812705-6.00005-8.

Die eigentliche Synthese der Rhamnolipide erfolgt auf biotechnologischen Wege mit Hilfe von Mikroorganismen, deren Stoffwechsel die Rhamnolipide zu bilden vermag. Dazu werden die Mikroorganismen in wässriger Umgebung mit einem Nährsubstrat gefüttert. Die Mikroorganismen setzen das Nährsubstrat in verschiedene Stoffwechselprodukte um, zu denen auch diverse Rhamnolipide nach Formel I gehören. Das Resultat der biotechnologischen Synthese ist eine so genannte Fermentationsbrühe. Dies ist ein komplexes Gemisch aus Wasser, Mikroorganismen, deren Stoffwechselprodukte und nicht umgesetzten Nährmedium. Die Herstellung der Fermentationsbrühe ist nicht Teil dieser Erfindung.

In einem zweiten Produktionsabschnitt gilt es nun, die Rhamnolipide aus der Fermentationsbrühe zu isolieren. Dies erfolgt mit unterschiedlichen Methoden der Verfahrenstechnik. Da die Fermentationsbrühe eine sehr komplexe Zusammensetzung aufweist und der Anteil der begehrten Rhamnolipiden an der Fermentationsbrühe lediglich 50 g/L bis 200 g/L beträgt, ist die Aufarbeitung der Fermentationsbrühe zwecks Gewinnung von reinen Rhamnolipiden aus eine äußerst anspruchsvolle verfahrenstechnische Aufgabe, insbesondere dann, wenn sie im industriellen Maßstab erfolgen soll. Tatsächlich werden die Produktionskosten und die Qualität, der in Wasch- und Reinigungsmitteln oder Kosmetika einsetzbaren Rhamnolipiden im Wesentlichen durch ihre Aufarbeitung bestimmt.

Anders als die biotechnologische Synthese ist die Aufarbeitung der Rhamnolipide kaum dokumentiert. Als Beleg des allgemeines Fachwissens kommt insbesondere die Darstellung von Weber und Ziemer in Betracht:
Andreas Weber and Tim Zeiner: Purification of Biosurfactants - Chapter 9 in: Biosurfactants. Edited By Naim Kosaric, Fazilet Vardar Sukan. 1st edition 2014 DOI 10.1201/b17599.

Ein industriell gut anwendbares Trennverfahren nutzt den Effekt, dass die Dichte der Rhamnolipide von dem pH-Wert des Umgebungsmedium abhängig ist: Durch gezielte Veränderung des pH-Wertes fallen die Rhamnolipide aus dem Medium aus oder lassen sich durch Phasentrennung abscheiden. Weber und Zeiner beschreiben dies in dem Abschnitt 9.3.6 PRECIPITATION/CRYSTALLIZATION.

Auch in der Patentliteratur ist dieses Vorgehen beschrieben: So offenbart die EP 2735605 B1 die Isolation von Rhamnolipid, bei dem der pH-Wert durch Zugabe einer mineralischen Säure gesenkt wird, sodass ein zweiphasiges Gemisch entsteht. Die schwere, rhamnolipidreiche Phase wird abgetrennt und anschließend durch Zugabe von Lauge wieder neutralisiert. Dieses Verfahren lässt sich im industriellen Maßstab durchführen. Nachteil ist jedoch, dass durch Zugabe von Säure und Lauge eine anorganische Salzfracht im Aufarbeitungsgemisch entsteht, die energiereich abgetrennt und entsorgt werden muss. Dadurch steigen die Produktionskosten. Ebenso nachteilig bei diesem Verfahren ist die Notwendigkeit, ein organisches Lösemittel wie etwa Ethylacetat zu nutzen. Dieses verursacht ebenfalls Kosten.

Abseits der Herstellung von Rhamnolipiden gab es bereits Versuche, die Aufarbeitung ähnlicher Verbindungen zumindest teilweise zu elektrifizieren:
So beschreibt etwa die CN 1199976 C die Verwendung einer bipolaren Membran zur elektrodialytischen Isolation von Zuckersäure. Da sich die Zusammensetzung der Fermentationsbrühe einer Rhamnolipidherstellung deutlich von dem Zuckersäuregemisch unterscheidet, ist dieses Verfahren nicht ohne Weiteres auf die Rhamnolipidherstellung übertragbar.

Zur Aufarbeitung einer Fermentationsbrühe nutzt die EP 3347481 B1 ebenfalls eine bipolare Membran. Ziel ist dabei aber nicht die Isolierung des Zielproduktes, sondern dessen Deionisierung.

Die Arbeitsgruppe um Chong Shen hat eine Elektrodialyse eingesetzt, um aus hydrolysierten Rhamnolipiden einen Tomatendünger herzustellen:
Chong Shen, Yizeng Li, Gang Lu, Qin Meng: / Electrodialysis treatment of rhamnolipids hydrolysate and its waste water for use as water-soluble fertilizer. Bioresource Technology, Volume 393, Year 2024, Article 130080 DOI 10.101 6/j.biortech.2023.130080.

Im Rahmen dessen wurden die Rhamnolipide erst mit Säure hydrolysiert, sodass Rhamnose mit pH 1.6 entsteht. Das Hydrolysat wurde anschließend in einer elektrochemischen Zelle auf pH 6.3 nahezu neutralisiert. Das so behandelte Hydrolysat enthält Rhamnose und keine Rhamnolipide. Es unterscheidet sich daher deutlich von der Fermentationsbrühe einer Rhamnolipidherstellung.

### Aufgabe

In Hinblick auf diesen Stand der Technik bestand die Aufgabe der Erfindung darin, ein Verfahren zur Isolation von Rhamnolipiden aus wässrigen Medien anzugeben, welches die Salzfracht reduziert und möglichst ohne organische Lösemittel auskommt. Zudem soll das Verfahren möglichst energieeffizient sein. Es soll Rhamnolipide mit hoher Reinheit liefern und sich im industriellen Maßstab anwenden lassen.

### Lösung

Gelöst wird diese Aufgabe durch ein Verfahren, welches die folgenden, nicht chronologischen Schritte aufweist:
a) Bereitstellen eines wässrigen Mediums, welches Wasser und mindestens ein Rhamnolipid enthält, wobei die Konzentration der Gesamtheit aller in dem wässrigen Medium enthaltener Rhamnolipide zwischen 50 g/L und 200 g/L beträgt, bezogen auf das Volumen des bereitgestellten wässrigen Mediums, und wobei der mit Glaselektrode bei 50°C gemessene pH Wert des wässrigen Mediums ein einem Bereich liegt, der sich von 5 bis 7 erstreckt;
b) Zugabe von Protonen H⁺ zu dem wässrigen Medium, sodass ein Zwischenprodukt erhalten wird, dessen mit Glaselektrode bei 50°C gemessener pH-Wert in einem Bereich liegt, der sich von 1 bis 4 oder von 2 bis 3 erstreckt,
c) Überführen des Zwischenprodukts in ein Gemisch, welches mindestens zwei Phasen umfasst, nämlich eine erste Phase mit der Dichte ρ₁ und eine zweite Phase mit der Dichte ρ₂, wobei Dichte der ersten Phase ρ₁ größer ist als die Dichte der zweiten Phase ρ₂ und wobei die erste Phase Wasser und mindestens ein Rhamnolipid enthält;
d) Phasentrennung des Gemisches, sodass die erste Phase und die zweite Phase getrennt erhalten werden;
e) Zugabe von Hydroxidionen OH⁻ zu der ersten getrennt erhaltenen Phase, sodass ein Produkt erhalten wird, dessen mit Glaselektrode bei 50°C gemessener pH-Wert in dem Bereich liegt, der sich von 5 bis 7 erstreckt, wobei das Produkt Wasser und mindestens ein Rhamnolipid enthält, und wobei die Konzentration der Gesamtheit aller in dem Produkt enthaltener Rhamnolipide zwischen 200 g/L und 800 g/L beträgt, bezogen auf das Volumen des Produkts;
f) Bereitstellen mindestens einer elektrochemischen Zelle, welche die folgenden Merkmale aufweist:
   α) die elektrochemische Zelle umfasst eine Anode;
   β) die elektrochemische Zelle umfasst eine Kathode;
   γ) die elektrochemische Zelle umfasst mindestens eine zwischen Anode und Kathode angeordnete Wiederholeinheit;
   δ) die Wiederholeinheit umfasst ein anodisches Kompartiment;
   ε) die Wiederholeinheit umfasst ein kathodisches Kompartiment;
   ζ) die Wiederholeinheit umfasst einen ionisch leitfähigen, elektrisch isolierenden Separator, welcher an das anodische Kompartiment und an das kathodische Kompartiment angrenzt;
   η) die Anode und die Kathode sind jeweils über eine jeweilige elektrische Leitung an eine elektrische Spannungsquelle angeschlossen, sodass die elektrochemische Zelle über die elektrischen Leitungen mit einer von der elektrischen Spannungsquelle bezogenen elektrischen Spannung beaufschlagbar ist;

wobei in dem erfindungsgemäßen Verfahren die Zugabe von Protonen H⁺ zu dem wässrigen Medium dadurch erfolgt, dass das kathodische Kompartiment der elektrochemischen Zelle mit dem wässrigen Medium beaufschlagt wird, währenddessen die elektrochemische Zelle mit einer von der elektrischen Spannungsquelle bezogenen elektrischen Spannung beaufschlagt wird,
wobei die Zugabe von Hydroxidionen OH⁻ zu der ersten getrennt erhaltenen Phase dadurch erfolgt, dass das anodische Kompartiment der elektrochemischen Zelle mit der getrennt erhaltenen ersten Phase beaufschlagt wird, währenddessen die elektrochemische Zelle mit der von der elektrischen Spannungsquelle bezogenen elektrischen Spannung beaufschlagt wird,
wobei das Zwischenprodukt aus dem kathodischen Kompartiment der elektrochemischen Zelle abgezogen wird,
und wobei das Produkt aus dem anodischen Kompartiment der elektrochemischen Zelle abgezogen wird.

Das erfindungsgemäße Verfahren nutzt das bewährte Absenken und Erhöhen des pH-Wertes, um die Rhamnolipide anhand ihrer Dichte abzuscheiden. Allerdings wird der pH-Wert elektrochemisch eingestellt, nämlich durch Elektrolyse von Wasser:
In der dafür eingesetzten elektrochemischen Zelle wird zwischen Anode und Kathode ein elektrisches Potential angelegt, welches das Wasser H₂O in Wasserstoffionen (Protonen) H⁺ und Hydroxidionen OH⁻spaltet. Die elektrochemische Zelle ist mit einer bipolaren Membran (BPM) oder alternativ mit einer Kombination aus einer Anionenaustauschmembran (AEM) und einer Kationenaustauschmembran (CEM) ausgerüstet. Die AEM bzw. der anodische Teil der BPM besitzt eine Leitfähigkeit für Hydroxidionen OH⁻, währenddessen die CEM bzw. der kathodische Teil der BPM eine Leitfähigkeit für Protonen H⁺ besitzt. Ihrer Polarität entsprechend wandern die Hydroxidionen OH⁻ durch die AEM bzw. anodischen Teil der BPM zur Anode, die Protonen H⁺ durch die CEM bzw. den kathodischen Teil der BPM zur Kathode.

Erfindungsgemäß ist die elektrochemische Zelle so konfiguriert, dass das wässrige, leicht saure (pH 5 bis 7) Ausgangsmedium als Katholyt im kathodischen Kompartiment der Zelle vorgelegt wird. Durch die Anreicherung der Protonen im kathodischen Kompartiment nimmt der pH-Wert des Katholyts ab; das Medium wird saurer (pH 3 bis 5), wodurch letztendlich das zweiphasige Gemisch entsteht, in dessen schwererer Phase sich die Rhamnolipide befinden.

Die schwerere Phase wird mit herkömmlichen Mitteln aus dem Gemisch abgetrennt. Dies geschieht außerhalb der elektrochemischen Zelle.

Die Erhöhung des pH-Werts auf den Zielwert erfolgt wieder in der elektrochemischen Zelle: Hierfür wird die schwere rhamnolipidhaltige Phase als Anolyt in das anodische Kompartiment gefahren. Dort reichern sich die Richtung Anode wandernden Hydroxidionen OH- im Anolyt an, sodass dessen pH-Wert wieder steigt. Mit dem zwischen 5 und 7 angestrebten pH wird der Anolyt wieder aus der Zelle abgezogen und stellt somit das Produkt des Verfahrens dar.

Dank der elektrodialytischen Aufkonzentrierung erreicht das Produkt eine Rhamnolipid-Konzentration von bis zu 800 g/L, was bei einer Dichte von 1.1 kg/l einer Massenkonzentration von 72 Gew.-% entspricht (hochkonzentriertes Produkt).

Ein wesentlicher Aspekt des vorliegenden Verfahrens ist demnach, dass sowohl die Absenkung, als auch die Erhöhung des pH-Wertes elektrochemisch durch Wasserspaltung erfolgt. Dies hat den entscheidenden Vorteil, dass keine anorganischen Säuren oder Laugen eingesetzt werden müssen. Die Salzfracht nimmt dadurch ab. Dadurch müssen weniger Salze destillativ abgetrennt werden. Die elektrische Energie, die für die pH-Einstellung benötigt wird, kann im Übrigen aus erneuerbaren Quellen gewonnen werden. Dies ist bei klassischer Destillation, die Wärmeenergie benötigt, nicht so ohne verlustreiche Energieumwandlung möglich. Deswegen erzielt das erfindungsgemäße Verfahren potentiell einen geringeren CO₂-Fußabdruck als ein herkömmliches Verfahren, bei dem ein Salz thermisch abdestilliert wird.

Ein weiterer Vorteil des Verfahrens ist auch, dass es sich ohne organische Lösemittel ausführen lässt.

Schließlich lassen sich elektrochemische Zellen durch Anpassung der Zellfläche und/oder durch Parallelisierung leicht skalieren. Auf diese Weise kann das Verfahren einfach in einem größeren Produktionsmaßstab angewandt werden.

Wie bereits erwähnt, wird im erfindungsgemäßen Verfahren eine Wasserspaltung in der Zelle durchgeführt. Die dabei entstehenden Hydroxidionen OH⁻ werden in dem anodischen Kompartiment angereichert, weil sie ihrer Ladung entsprechend zur Anode wandern. Die Protonen H⁺ reichern sich in dem kathodischen Kompartiment an, weil es sie zur Kathode zieht.

Die für die Veränderung des pH-Wertes notwendigen Protonen H⁺ werden also aus der Wasserelektrolyse gewonnen. Die Wasserelektrolyse findet vorzugsweise mit Hilfe einer bipolaren Membran (BPM) statt, die als Separator genutzt wird. Eine BPM ist formal eine Kombination aus einer Anionenaustauschmembran (AEM) und einer Kationenaustauschmembran (CEM). Sie besteht aus einem positiv geladenen Teil zum Leiten der Anionen und einen negativ geladenen Teil zum Leiten der Kationen. Das Wasser dringt in die Membran ein und wird an der Grenzschicht zwischen AEM und CEM getrennt. Die Wasserspaltung mit Hilfe einer BPM ist bewährte Technologie. Geeignete bipolare Membranen sind kommerziell erhältlich.

Eine bevorzugte Weiterbildung der Erfindung sieht vor, dass die elektrochemische Zelle eine erste Spülkammer aufweist. Die Spülkammer wird zwischen Wiederholeinheit und Anode angeordnet und durch eine zugeordnete Kationenaustauschmembran von der Wiederholeinheit abgegrenzt. Die Spülkammer wird von einer ersten Spüllösung beaufschlagt. Die Spülkammer dient dazu, Nebenprodukte des elektrochemischen Verfahrens, nämlich Sauerstoff und ggf. auch Lauge aus der Zelle abzuführen. Als Spüllösung wird ein wässriger Elektrolyt verwendet, zum Beispiel eine leichte Salzlösung.

Analog kann auch die kathodische Seite der elektrochemischen Zelle mit einer Spülkammer ausgerüstet sein. Die zweite Spülkammer dient zum Abführen von Säure und Wasserstoff, was beides kathodenseitig als Nebenprodukt entsteht. Die zweite Spülkammer wird auch durch eine zweite Kationenaustausch-membran von der Wiederholeinheit abgegrenzt. Die Spüllösung sollte wieder ein wässriger Elektrolyt sein.

Vorzugsweise wird für beide Spülkammern die identische Spüllösung verwendet. Dies macht den Prozess einfacher. Außerdem können beide Spüllösungen durch ein gemeinsames Gefäß rezykliert werden, was die Neutralisation von entstehender Lauge und Säure gestattet. Als Spüllösung eignet sich beispielsweise wässrige Natriumsulfat-Lösung. Der Salzgehalt sollte so gering wie möglich sein, etwa 5 Gew.-% bezogen auf die Spüllösung genügen bei der Nutzung von Natriumsulfat.

Das Verfahren lässt sich einfach dadurch skalieren, dass die Anzahl der Wiederholeinheiten innerhalb der Zelle erhöht wird. Vorzugsweise weist die elektrochemische Zelle mindestens zwei Wiederhol-einheiten auf. Die beiden Wiederholeinheiten werden durch eine dritte Kationenaustausch-membran voneinander abgegrenzt. Jede weitere Wiederholeinheit wird wiederum durch eine zusätzliche Kationenaustauschmembran von der benachbarten Wiederholeinheit abgrenzt. Mit der Anzahl der Wiederholeinheiten erhöht sich die Kapazität der Zelle und damit der mit dem Verfahren mögliche Durchsatz. Die Anzahl der Separatoren und Kationenaustauschmembranen steigt mit der Anzahl Wiederholeinheiten. Der mit der Anzahl der Membranen anwachsende Innenwiderstand der Zelle begrenzt die Anzahl realisierbaren Wiederholeinheiten. Die Anzahl der beiden Elektroden ändert sich bei Erhöhung der Wiederholeinheiten indes nicht.

Das als Katholyt aus der Zelle abgezogene, saure Zwischenprodukt ist in ein zweiphasiges Gemisch zu überführen, was anschließend einer Phasentrennung unterzogen wird. Idealerweise wird das Zwischenprodukt direkt als Gemisch genutzt. Das ist in der Praxis allerdings in vielen Fällen nicht möglich. Vielmehr ist es technisch sinnvoll, das Zwischenprodukt einer Waschung zu unterziehen, um Störstoffe, insbesondere mineralische Salze abzutrennen. Ebenso ist es sinnvoll, das Zwischenprodukt abzukühlen, weil sich dann die Phasen besser voneinander trennen lassen. Vorzugweise erfolgt die Überführung des Zwischenprodukts in das Gemisch daher durch Waschen und/oder durch Abkühlen. Beides kann mit üblichen verfahrenstechnischen Operationen erfolgen. Das Abkühlen kann auch durch das Waschen erfolgen. Waschen und Abkühlen wird dann in einem Arbeitsschritt erledigt.

Das Verfahren wird optimalerweise in einem Betriebstemperaturbereich ausgeführt, der sich innerhalb von 20°C bis 70°C erstreckt. So kann sich der Betriebstemperaturbereich beispielweise von 40°C bis 60°C erstrecken. Dies bedeutet, dass alle verarbeiteten Stoffe eine Temperatur in dem genannten Bereich aufweisen, und dass die Apparate entsprechend temperiert sind, um diese Stofftemperaturen zu gewährleisten. Zumindest sollte mindestens eine, besser noch mehre der folgenden Temperaturen im genannten Betriebstemperaturbereich liegen:
- Temperatur des wässrigen Mediums;
- Temperatur des Zwischenprodukts;
- Temperatur des Gemisches;
- Temperatur der getrennt erhaltenen ersten Phase.

Diese Temperaturen brauchen nicht alle gleich zu sein. Insbesondere kann das Gemisch kühler sein als das Zwischenprodukt, nämlich dann, wenn die Überführung des Zwischenprodukt in das Gemisch zwecks Vereinfachung der Phasentrennung mit einer Kühlung einhergeht.

Da das Produkt des Verfahrens mit der Zeit Umgebungstemperatur annimmt, sei klargestellt, dass sich die interessierende Temperatur des Produkts auf den Zeitpunkt unmittelbar nach Erhalt des Produkts bezieht. Zumindest zu diesem Zeitpunkt sollte die Temperatur des Produkts im Betriebstemperaturbereich liegen; später, nach dem Abzug aus dem ersten Kompartiment der Zelle kann das Produkt abkühlen. Der Grund dafür ist, dass die erste, schwere rhamnolipidreiche Phase, die als Anolyt verwendet wird, bei niedrigen Temperaturen dickflüssig wird und sich dann schwieriger handhaben lässt. Vorzugsweise wird die erste Phase vor dem Eintritt in das anodische Kompartiment aufgewärmt, damit es dieses nicht zusetzt. Eine Erwärmung ist insbesondere dann notwendig, wenn das Zwischenprodukt zwecks Phasentrennung zuvor abgekühlt wurde. Die bei der Abkühlung des Zwischenproduktes gewonnene Wärmeenergie wird vorzugsweise zur Erwärmung der ersten Phase verwendet. Auf diese Weise wird Energie eingespart.

Die zweite, leichte Phase wird nach der Phasentrennung aus dem Prozess ausgeschleust. Sie enthält keine Wertprodukte.

Das wässrige Medium, welches als Ausgangstoff für das erfindungsgemäße Verfahren dient, stammt ursprünglich aus einer nicht zur Erfindung gehörenden Fermentation, in der die Rhamnolipide biotechnologisch hergestellt werden. Da die dabei erhaltene Fermentationsbrühe wird in der Regel vor dem Eintritt in das kathodische Kompartiment mit herkömmlichen verfahrenstechnischen Mitteln aufgereinigt werden müssen, weil die Fermentationsbrühe noch zu viele Störstoffe enthält. Auch eine Entschäumung kann erforderlich sein. Folglich wird das wässrige Medium durch an sich bekanntes Aufarbeiten aus der Fermentationsbrühe erhalten.

Die Fermentationsbrühe enthält vor ihrer Aufarbeitung zum wässrigen Medium zumindest einen Mikroorganismus, welcher die Rhamnolipide gebildet hat. Zumindest diese Mikroorganismen müssen im Rahmen der Aufarbeitung abgetrennt werden. Bei besagten Mikroorganismen handelt es sich um Vertreter der *Actinobacteria* oder *Bacillota* oder *Pseudomonadota,* insbesondere *um Pseudomonas aeruginosa* oder um *Pseudomonas alloputida* oder um *Pseudomonas putida.* Diese Arten werden üblicherweise zur biotechnologischen Herstellung der Rhamnolipide eingesetzt.

Ein Vorteil des elektrochemischen Verfahrens ist, dass es ohne organische Hilfsmedien auskommt. In herkömmlichen Trennverfahren werden nämlich organische Hilfsmedien wie etwa Methylacetat, Ethylacetat, Propylacetat, Isopropylacetat, Butylacetat, Isoamylacetat, Butan-1-ol, Butan-2-ol, Diethylether oder Mischungen daraus eingesetzt. Dies ist hier nicht notwendig. Es ist aber nicht ausgeschlossen, dass diese Hilfsmedien im Zuge der Aufarbeitung der Fermentationsbrühe genutzt werden müssen. In dem Fall sollte das Hilfsmedium aber vor der elektrochemischen Abtrennung der Rhamnolipide aus dem Stoffstrom wieder entfernt werden, sodass der Gehalt des organischen Hilfsmediums im wässrigen Medium kleiner ist als 5 Gew.-%. Der Gewichtsanteil bezieht sich auf das Gewicht des wässrigen Mediums. Sofern mehre organische Substanzen als Hilfsmedium eingesetzt wurden, gilt die Obergrenze von 5 Gew.-% für die Summe der Anteile aller Substanzen, die als organisches Hilfsmedium in Betracht kommen.

Ganz besonders bevorzugt wird abgesehen von der Aufarbeitung der Fermentationsbrühe keine Substanz eingesetzt, die als organisches Hilfsmedium in Betracht kommt.

### Figurenbeschreibung

Die Erfindung soll nun anhand von Verfahrensfließbildern näher erläutert werden. Hierfür zeigen:
- Figur 1:: Verfahrensfließbild mit schematisch dargestellter, erster Ausführungsform einer elektrochemischen Zelle;
- Figur 2:: Verfahrensfließbild mit schematisch dargestellter, zweiter Ausführungsform einer elektrochemischen Zelle;
- Figur 3:: Verfahrensfließbild mit schematisch dargestellter, dritter Ausführungsform einer elektrochemischen Zelle.

Ausgangspunkt ist die Bereitstellung eines wässrigen Mediums 0 enthaltend Rhamnolipide. Bei dem wässrigen Medium kann es sich beispielsweise um die Fermentationsbrühe handeln, die aus dem eigentlichen Bioreaktor (nicht dargestellt) abgezogen worden ist. Es kann sich aber auch um das Resultat einer oder mehrerer Aufarbeitungsschritte handeln, welche auf die Fermentationsbrühe angewandt worden sind, etwa Entschäumung oder Entfernung der Biomasse durch Filtrierung.

Das wässrige Medium 0 enthält im Wesentlichen flüssiges Wasser. Spezifikationsgemäß ist der Anteil an Rhamnolipiden mit 50 bis 200 g/L recht klein, was auf dem Gebiet der Herstellung von Biotensiden aber nicht ungewöhnlich ist. Daneben kann das wässrige Medium 0 noch weitere Stoffe enthalten, muss es aber nicht. Insbesondere können nicht abgetrennte Feststoffe und auch Gase enthalten sein. Da bei Fermentation in der Regel Wärme entsteht, ist das wässrige Medium 0 entsprechend warm, etwa 30°C. Erforderlichenfalls wird es noch erwärmt, sodass es im gewünschten Betriebstemperaturbereich liegt. Das wässrige Medium 0 ist leicht sauer, der bei 50°C gemessene pH Wert liegt spezifikationsgemäß in einem Bereich von 5 bis 7. Die genannte Referenztemperatur von 50°C muss nicht notwendigerweise im gewählten Betriebstemperaturbereich liegen, gleichwohl ist das aber sinnvoll, weil so die Überwachung des pH-Wertes vereinfacht wird.

Zwecks Absenkung des pH-Wertes wird das wässrige Medium 0 in eine elektrochemische Zelle 1 gefahren.

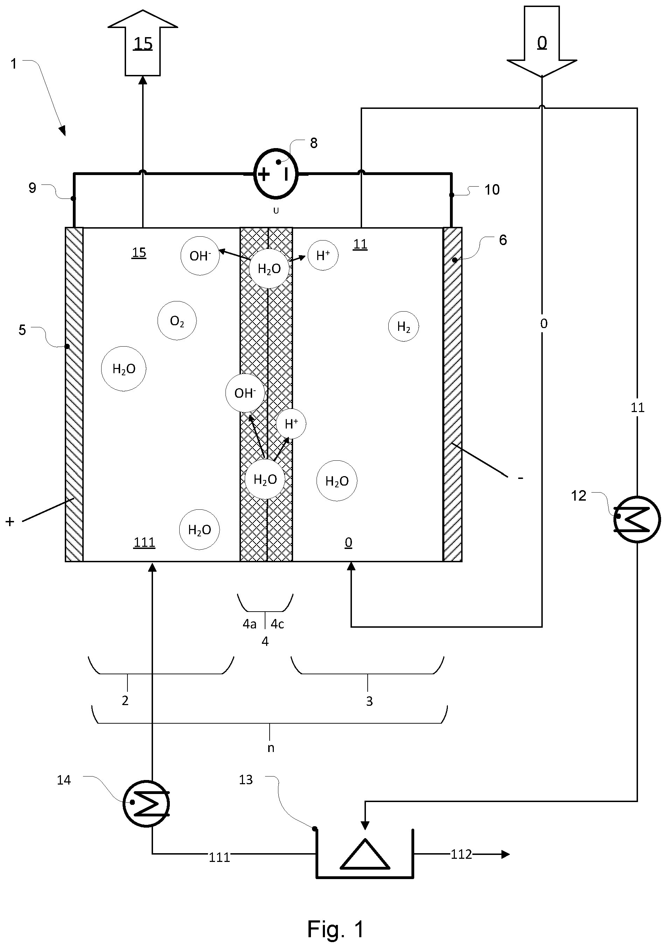

Die in Figur 1 dargestellte, erste Ausführungsform der elektrochemischen Zelle 1 umfasst genau zwei Kompartimente, nämlich ein erstes Kompartiment 2 und ein zweites Kompartiment 3. Die beiden Kompartimente 2, 3 sind durch genau einen Separator 4 voneinander getrennt. Der Separator 4 grenzt bei der in Figur 1 dargestellten ersten Ausführungsform an beide Kompartimente 2, 3 an. Erforderlichenfalls können beide Kompartimente 2, 3 jeweils von einem Spacer offen gehalten werden (nicht dargestellt). Bei dem Separator 4 handelt es sich um eine bipolare Membran, die sich wiederum formal aus einer Anionenaustauschmembran 4a und einer Kationenaustauschmembran 4c zusammensetzt. Die Anionenaustauschmembran 4a ist in Richtung des ersten Kompartiments 2 angeordnet, die Kationenaustauschmembran 4c in Richtung des zweiten Kompartiments 3.

Die beiden Kompartimente 2, 3 und der Separator 4 bilden zusammen eine Wiederholeinheit n. Die Anzahl der Wiederholeinheiten n in der in Figur 1 dargestellten Ausführungsform beträgt mithin eins.

Die Wiederholeinheit n ist beidseitig von jeweils einer Elektrode 5, 6 begrenzt, nämlich eine Anode 5 und eine Kathode 6. Die Anode 5 grenzt an das erste Kompartiment 2, die Kathode 6 an das zweite Kompartiment. Das erste Kompartiment 2 wird daher hier als anodisches Kompartiment 2 bezeichnet, das zweite Kompartiment 3 als kathodisches Kompartiment 3.

Die elektrochemische Zelle 1 ist an eine elektrische Spannungsquelle 8 angeschlossen. Dafür ist die Anode 5 über eine erste elektrische Leitung 9 mit dem Pluspol der elektrischen Spannungsquelle 8 verbunden, der Minuspol ist über eine zweite elektrische Leitung 10 mit der Kathode 6 kontaktiert. Die von der elektrischen Spannungsquelle 8 bezogene elektrische Spannung U baut einen entsprechenden Potentialunterschied zwischen Anode 5 und Kathode 6 auf.

Um den pH-Wert des wässrigen Mediums 0 abzusenken, wird dieses in das kathodische Kompartiment 3 gefahren. Das wässrige Medium 0 bildet dort den Katholyt. Das in dem wässrige Medium 0 enthaltene Wasser H₂O dringt durch die Kationenaustauschmembran 4c in den Separator 4 ein und wird darin durch die zwischen Anode 2 und Kathode 3 wirkende Spannung U gespalten. Die dabei einstehenden Protonen H⁺ verlassen den Separator 4 über die Kationenaustauschmembran 4c und wandern ihrem Ladungszustand entsprechend Richtung Kathode 6. Dabei reichern sie sich in dem wässrigen Medium 0 an, sodass dessen pH auf einen Wert im Bereich von 2 bis 3 sinkt (bei 50°C gemessen). Auf diese Weise wird ein Zwischenprodukt 11 erhalten, welches aus dem kathodischen Kompartiment 3 abgezogen wird. Außerdem bildet sich an der Kathode gasförmiger Wasserstoff H₂.

Aufgrund des abgesenkten pH Wertes bilden sich in dem Zwischenprodukt 11 zwei Phasen aus, eine erste schwerere Phase 111, welche die Rhamnolipide enthält und eine zweite, leichtere Phase 112, welche im Wesentlichen Wasser enthält. Die Konzentration der Rhamnolipide in der ersten Phase 111 beträgt etwa 250 bis 750 g/l und ist daher deutlich höher als im ursprünglich bereit gestellten wässrigen Medium 0. Die Dichte ρ₁ der ersten Phase 111 ist aufgrund des hohen Rhamnolipidgehaltes größer als die Dichte ρ₂ der zweiten Phase 112. Um die Phasentrennung zu erleichtern, wird das Zwischenprodukt 11 in einem ersten Wärmetauscher 12 etwas abgekühlt ohne den vorgesehenen Betriebstemperaturbereich zu verlassen. Das nunmehr gekühlte Gemisch wird Mithilfe einer Zentrifuge 13 eine Phasentrennung vollzogen, sodass die erste Phase 111 und die zweite Phase 112 getrennt erhalten werden. Die zweite, leichtere Phase 112 enthält keine Wertstoffe und wird ausgeschleust.

Die erste, schwerere Phase 111 enthaltend die Rhamnolipide wird nun zunächst in einem zweiten Wärmetauscher 14 wieder erwärmt und in als Anolyt in das anodische Kompartiment 2 der elektrochemischen Zelle 1 gefahren. Dort reichern sich in der ersten Phase 111 Hydroxidionen OH⁻ an, welche ebenfalls in der Wasserspaltung entstehen und durch die Anionenaustauschmembran 4a in Richtung der Anode 5 wandern. Durch die Anreichung der Hydroxidionen OH⁻ im Anolyt steigt dessen pH-Wert auf 5 bis 7. Daneben entsteht an der Anode gasförmiger Sauerstoff O₂.

Auf diese Weise wird ein Produkt 15 erhalten, welches aus dem anodischen Kompartiment 2 abgezogen wird. Das Produkt 15 enthält die begehrten Rhamnolipide in hoher Reinheit und Konzentration bei schwach saurem pH-Wert.

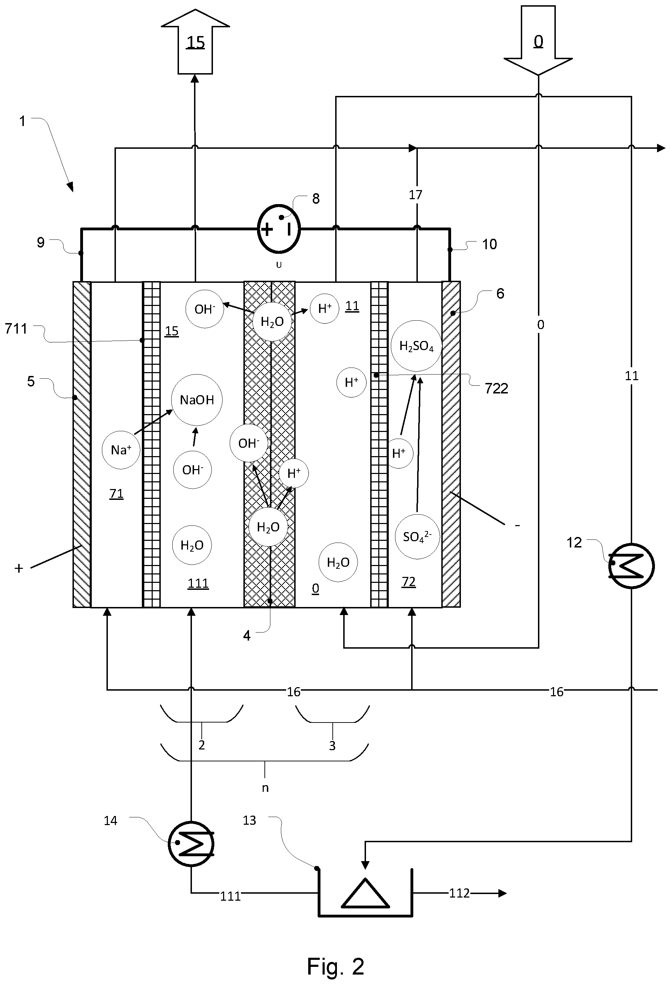

Figur 2 zeigt eine zweite Ausführungsform der elektrochemischen Zelle 0. Bei dieser Ausführungsform befindet sich zwischen Anode 5 und Wiederholeinheit n eine erste Spülkammer 71. Die erste Spülkammer 71 ist durch eine erste Kationenaustauschmembran 711 von der Wiederholeinheit n, genauer gesagt, von deren ersten (anodischen) Kompartiment 2 abgegrenzt. Analog grenzt eine zweite Kationenaustauschmembran 722 eine zweite Spülkammer 72 von dem zweiten (kathodischen) Kompartiment 3 ab. Lateral grenzt die zweite Spülkammer 72 an die Kathode 6.

Die beiden Spülkammern 71, 72 dienen dazu, die Elektroden 5, 6 zu spülen. Dazu werden beide Spülkammern 71, 72 jeweils mit derselben frischen Spüllösung 16 beaufschlagt. Dabei kann es sich beispielsweise um 5wt%-Natriumsulfat-Lösung handeln. Das Natriumsulfat ist enthalten, um die Leitfähigkeit der Spüllösung sicherzustellen. Mit der Spüllösung werden insbesondere die bei der Wasserelektrolyse entstehenden Gase Wasserstoff H₂ und Sauerstoff O₂ aus der Zelle abtransportiert. Der Wasserstoff entsteht an der Kathode, der Sauerstoff an der Anode. Wenn diese Gase nicht über die Spüllösung abtransportiert würden, könnte der Druck in der Zelle ungebührlich steigen. Darüber hinaus dient die Spüllösung dazu, die aus dem Natriumsulfat an der Anode entstehende Lauge und die an der Kathode entstehende Säure anzuführen und gegeneinander zu neutralisieren. Bei der Lauge handelt es sich um Natriumhydroxid NaOH, was aus dem Na⁺ und dem OH⁻ an der Anode entsteht. Bei der Säure handelt es sich um Schwefelsäure H₂SO₄, welche an der Kathode gebildet wird. Durch Zusammenführung der beiden Spüllösungen neutralisieren sich Schwefelsäure und Natriumhydroxid, wobei wieder Natriumsulfat entsteht (nicht dargestellt). Der Salzkreislauf ist daher im System geschlossen.

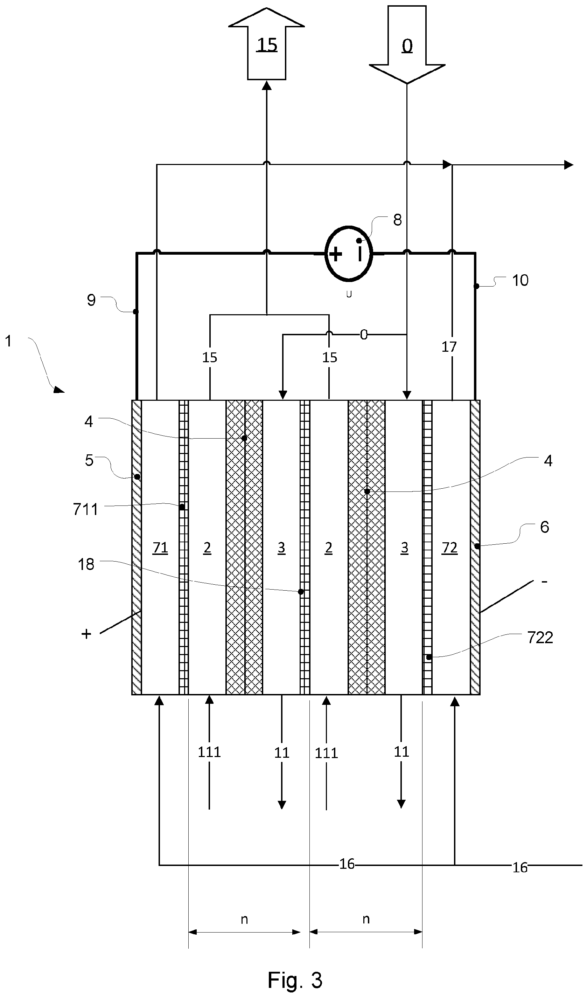

Figur 3 zeigt eine weitere Ausführungsform der Erfindung. Diese unterscheidet sich von den in Figur 1 und 2 dargestellten Zellen dadurch, dass sie genau zwei Wiederholeinheiten enthält. Die beiden Wiederholeinheiten n sind von einer dritten Kationenaustauschmembran 18 voneinander abgegrenzt.

Die Verdoppelung der Wiederholeinheiten n bewirkt zunächst, dass die Anzahl der Separatoren 4 und die Anzahl der Kompartimente 2, 3 sich ebenfalls verdoppeln. Dadurch verdoppelt sich wiederum die zur Verfügung stehende Zellfläche, weswegen die elektrochemische Zelle 0 eine entsprechend höhere Kapazität hat. Die Erhöhung der Anzahl der Wiederholeinheiten stellt damit eine Maßnahme zur Skalierung der Leistung der elektrochemischen Zelle 0, genauer gesagt, eine Parallelisierung.

Die doppelten Kompartimente 2, 3 werden jeweils parallel mit wässriger Lösung 0 (im Fall der kathodischen Kompartimente 3) bzw. mit der getrennt erhaltenen ersten Phase 111 (im Fall der beiden anodischen Kompartimente 2) beaufschlagt.

Ebenfalls parallel beaufschlagt werden die beiden bei der in Figur 3 dargestellten Ausführungsform auch vorhandenen Spülkammern 71, 72 mit frischer Spüllösung 16.

Durch diesen geschilderten Aufbau ergibt sich innerhalb der in Figur 3 dargestellten elektrochemischen Zelle 0 folgender Stapelaufbau (Stack):
- Anode 5
- erste Spülkammer 71
- erste Kationenaustauschmembran 711
- anodisches Kompartiment 2 der ersten Wiederholeinheit
- Separator 4 der ersten Wiederholeinheit
- kathodisches Kompartiment 3 der ersten Wiederholeinheit
- dritte Kationenaustauschmembran 18
- anodisches Kompartiment 2 der zweiten Wiederholeinheit
- Separator 4 der zweiten Wiederholeinheit
- kathodisches Kompartiment 3 der zweiten Wiederholeinheit
- zweite Kationenaustauschmembran 722
- zweite Spülkammer 72
- Kathode 6

### Beispiele

Die von dem erfindungsgemäßen Verfahren genutzte elektrochemische Einstellung des pH-Werts soll nun anhand von Beispielen belegt werden.

Der Versuchsaufbau arbeitet mit der in Figur 3 dargestellten Ausführungsform einer elektrochemischen Zelle.

Allerdings sieht der Versuchsaufbau einen Batch-Betrieb vor: Demnach werden die Versuchslösungen in Doppelwandbehältern vorgehalten und mit Pumpen umgewälzt. Das in den Behältern vorgelegte Batch wird in mehreren Durchläufen verarbeitet. Anolyt und Katholyt sind im Versuch getrennt und nicht in Serie geführt.

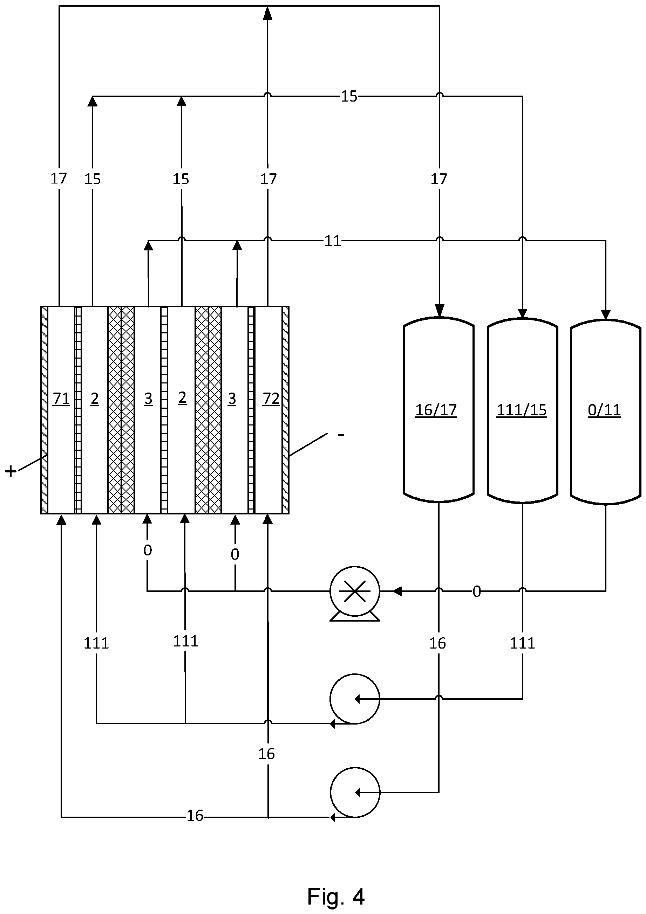

### Figur 4: Versuchsaufbau

Die rechts in Figur 4 dargestellte Elektrodialysezelle befindet sich in einem auf 60°C beheizten Klimaschrank.

Die folgenden Geräte und Materialien wurden eingesetzt:
- Eingesetzte Elektrodialyse-Zelle: Deukum, 320 Quadro
- Eingesetzte Membranen:
- 3x CEM von Reichelt Typ: RCT-High-Mechanical-Strength. CMX;
- 2x BPM von Lanran Typ BP-2
- Elektroden aus Stahl und DSA
- Eingesetzte Pumpen: Schlauchpumpe für Anolyt, Kreiselpumpe für Katholyt und Kreiselpumpe für Elektrodenspülung
- Anolyt: 0,65 L Rhamnolipid, pH 3,0 (schwere Phase)
- Katholyt: 2,6 L Rhamnolipid, pH 5,0
- Elektrodenspüllösung: 1 L 5w% Na₂SO₄
- Erwärmung alle Stoffströme auf ca. 50°C über Doppelmantelbehälter und Thermostat
- Klimaschrank zur Erwärmung der zusammengebauten Elektrodialysezelle
- Inertisierung des Klimaschranks mit Stickstoff

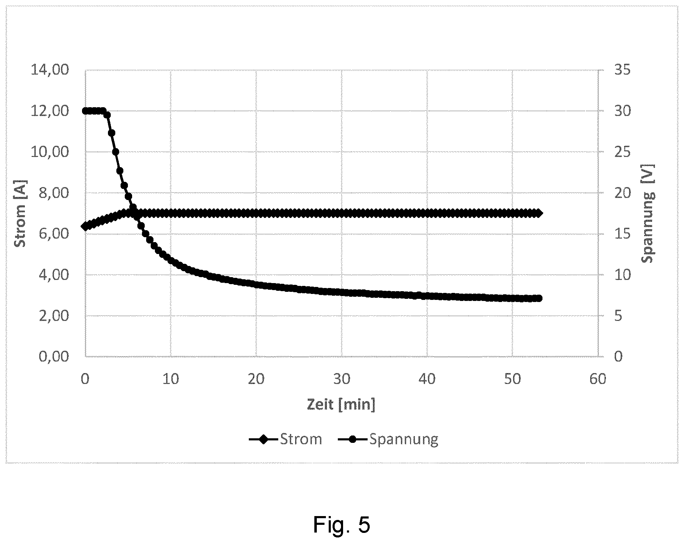

Strom und Spannung im Versuchsverlauf ist in Figur 5 dargestellt.

### Figur 5: Strom und Spannung im Versuchsverlauf

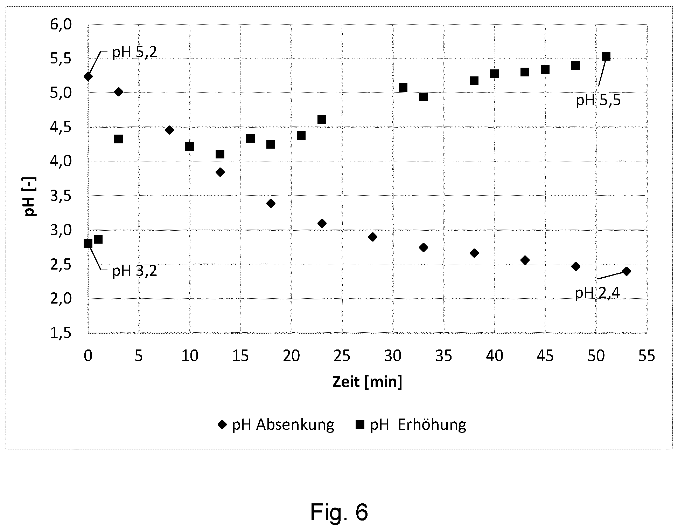

Die pH Werte der beiden Elektrolytströme im Versuchsverlauf sind in Figur 6 dargestellt:

### Figur 6: pH-Werte der beiden Elektrolytströme im Versuchsverlauf

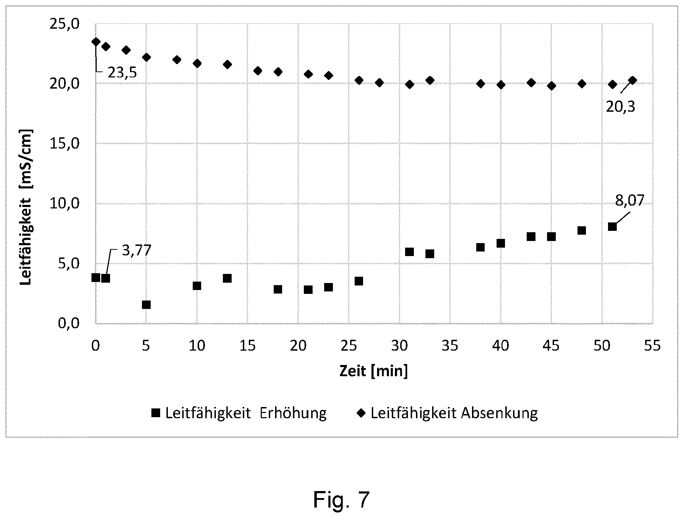

Die elektrische Leitfähigkeit der beiden Elektrolytströme im Versuchsverlauf sind in Figur 7 dargestellt:

### Figur 7: elektrische Leitfähigkeit der beiden Elektrolytströme im Versuchsverlauf

Anhand der Figur 6 ist zu erkennen, dass sich der pH-Wert der beiden Ströme tatsächlich elektrochemisch einstellen lässt.

Wie aus dem Stand der Technik bekannt, bewirkt die Absenkung des pH-Wertes eine Phasentrennung, sodass sich die Rhamnolipid-reiche Phase aus dem Gemisch abtrennen lässt. Die Abtrennung kann in herkömmlicher Weise erfolgen. Die Versuche belegen, dass sich der pH-Wert der Rhamnolipid-reichen Phase danach wieder auf den gewünschten Wert des Endprodukts anheben lässt.

Auf Grundlage dieser Erkenntnis lässt sich das in EP2735605 beschriebene Verfahren dahingehend abwandeln, dass die Absenkung und Anhebung des pH-Wertes wie hier beschrieben elektrochemisch erfolgt. Dies reduziert den Einsatz von Säure und Lauge erheblich wodurch die Salzfracht sinkt. Lediglich das Salz für die Spüllösung ist noch erforderlich. Der Bedarf an Wärmeenergie zur Entfernung der Salzfracht wird dadurch deutlich reduziert.

### Bezugszeichenliste

- 0: wässriges Medium
- 1: elektrochemische Zelle
- 2: anodisches Kompartiment
- 3: kathodisches Kompartiment
- 4: Separator (bipolare Membran)
- 4a: Anionenaustauschmembran (Teil der bipolaren Membran)
- 4c: Kationenaustauschmembran (Teil der bipolaren Membran)
- 5: Anode
- 6: Kathode
- 71: erste Spülkammer
- 711: erste Kationenaustauschmembran
- 72: zweite Spülkammer
- 722: zweite Kationenaustauschmembran
- 8: elektrische Spannungsquelle
- 9: erste elektrische Leitung
- 10: zweite elektrische Leitung
- 11: Zwischenprodukt
- 111: erste Phase
- 112: zweite Phase
- 12: erster Wärmetauscher
- 13: Zentrifuge
- 14: zweiter Wärmetauscher
- 15: Produkt
- 16: Spüllösung frisch
- 17: Spüllösung verunreinigt
- 18: dritte Kationenaustauschmembran

- U: Spannung
- n: Wiederholeinheit

- H₂O: Wasser
- H⁺: Protonen
- OH⁻: Hydroxidionen
- Na⁺: Natrium-Kationen
- SO₄²⁻: Sulfat-Anionen
- H₂: Wasserstoff
- O₂: Sauerstoff
- NaOH: Natriumhydroxid
- H₂SO₄: Schwefelsäure

## Patentansprüche

1. Verfahren zur Isolation von Rhamnolipiden aus wässrigen Medien, welches die folgenden, nicht chronologischen Schritte aufweist:
a) Bereitstellen eines wässrigen Mediums, welches Wasser und mindestens ein Rhamnolipid enthält, wobei die Konzentration der Gesamtheit aller in dem wässrigen Medium enthaltener Rhamnolipide zwischen 50 g/L und 200 g/L beträgt, bezogen auf das Volumen des bereitgestellten wässrigen Mediums, und wobei der mit Glaselektrode bei 50°C gemessene pH Wert des wässrigen Mediums ein einem Bereich liegt, der sich von 5 bis 7 erstreckt;
b) Zugabe von Protonen H⁺ zu dem wässrigen Medium, sodass ein Zwischenprodukt erhalten wird, dessen mit Glaselektrode bei 50°C gemessener pH-Wert in einem Bereich liegt, der sich von 1 bis 4 oder von 2 bis 3 erstreckt,
c) Überführen des Zwischenprodukts in ein Gemisch, welches mindestens zwei Phasen umfasst, nämlich eine erste Phase mit der Dichte ρ₁ und eine zweite Phase mit der Dichte ρ₂, wobei Dichte der ersten Phase ρ₁ größer ist als die Dichte der zweiten Phase ρ₂ und
wobei die erste Phase Wasser und mindestens ein Rhamnolipid enthält;
d) Phasentrennung des Gemisches, sodass die erste Phase und die zweite Phase getrennt erhalten werden;
e) Zugabe von Hydroxidionen OH⁻ zu der ersten getrennt erhaltenen Phase, sodass ein Produkt erhalten wird, dessen mit Glaselektrode bei 50°C gemessener pH-Wert in dem Bereich liegt, der sich von 5 bis 7 erstreckt, wobei das Produkt Wasser und mindestens ein Rhamnolipid enthält, und wobei die Konzentration der Gesamtheit aller in dem Produkt enthaltener Rhamnolipide zwischen 200 g/L und 800 g/L beträgt, bezogen auf das Volumen des Produkts;
**dadurch gekennzeichnet, dass** das Verfahren zusätzlich den folgenden Schritt umfasst:
f) Bereitstellen mindestens einer elektrochemischen Zelle, welche die folgenden Merkmale aufweist:
α) die elektrochemische Zelle umfasst eine Anode;
β) die elektrochemische Zelle umfasst eine Kathode;
γ) die elektrochemische Zelle umfasst mindestens eine zwischen Anode und Kathode angeordnete Wiederholeinheit;
δ) die Wiederholeinheit umfasst ein anodisches Kompartiment;
ε) die Wiederholeinheit umfasst ein kathodisches Kompartiment;
ζ) die Wiederholeinheit umfasst einen ionisch leitfähigen, elektrisch isolierenden Separator, welcher an das anodische Kompartiment und an das kathodische Kompartiment angrenzt;
η) die Anode und die Kathode sind jeweils über eine jeweilige elektrische Leitung an eine elektrische Spannungsquelle angeschlossen, sodass die elektrochemische Zelle über die elektrischen Leitungen mit einer von der elektrischen Spannungsquelle bezogenen elektrischen Spannung beaufschlagbar ist;
wobei die Zugabe von Protonen H⁺ zu dem wässrigen Medium dadurch erfolgt, dass das kathodische Kompartiment der elektrochemischen Zelle mit dem wässrigen Medium beaufschlagt wird, währenddessen die elektrochemische Zelle mit einer von der elektrischen Spannungsquelle bezogenen elektrischen Spannung beaufschlagt wird,
wobei die Zugabe von Hydroxidionen OH⁻ zu der ersten getrennt erhaltenen Phase dadurch erfolgt, dass das anodische Kompartiment der elektrochemischen Zelle mit der getrennt erhaltenen ersten Phase beaufschlagt wird, währenddessen die elektrochemische Zelle mit der von der elektrischen Spannungsquelle bezogenen elektrischen Spannung beaufschlagt wird,
wobei das Zwischenprodukt aus dem kathodischen Kompartiment der elektrochemischen Zelle abgezogen wird,
und wobei das Produkt aus dem anodischen Kompartiment der elektrochemischen Zelle abgezogen wird.

2. Verfahren nach Anspruch 1, **dadurch gekennzeichnet, dass** es sich bei dem Separator um eine bipolare Membran handelt bzw. um eine Kombination aus einer Anionenaustauschmembran und einer Kationenaustauschmembran.

3. Verfahren nach Anspruch 1 oder 2, **dadurch gekennzeichnet, dass** die elektrochemische Zelle eine zwischen Wiederholeinheit und Anode angeordnete erste Spülkammer aufweist, dass die elektrochemische Zelle eine zwischen der ersten Spülkammer und der Wiederholeinheit angeordnete erste Kationenaustauschmembran umfasst, dass eine erste Spüllösung bereitgestellt wird, und dass die erste Spülkammer mit der ersten Spüllösung beaufschlagt wird.

4. Verfahren nach Anspruch 1 oder 2 oder 3, **dadurch gekennzeichnet, dass** die elektrochemische Zelle eine zwischen Wiederholeinheit und Kathode angeordnete zweite Spülkammer aufweist, dass die elektrochemische Zelle eine zwischen der zweiten Spülkammer und der Wiederholeinheit angeordnete zweite Kationenaustauschmembran umfasst, dass eine zweite Spüllösung bereitgestellt wird, und dass die zweite Spülkammer mit der zweiten Spüllösung beaufschlagt wird.

5. Verfahren nach den Ansprüchen 3 und 4, **dadurch gekennzeichnet, dass** erste und die zweite Spüllösung identisch sind.

6. Verfahren nach einem der Ansprüche 1 bis 5, **dadurch gekennzeichnet, dass** die elektrochemische Zelle mindestens zwei Wiederholeinheiten umfasst, wobei die beiden Wiederholeinheiten durch eine dritte Kationenaustauschmembran voneinander abgegrenzt sind.

7. Verfahren nach einem der vorhergehenden Ansprüche, **dadurch gekennzeichnet, dass** das Überführen des Zwischenproduktes in das Gemisch durch Abkühlen und/oder durch Waschen erfolgt.

8. Verfahren nach einem der vorhergehenden Ansprüche, wobei für das Verfahren ein Betriebstemperaturbereich bestimmt ist, welcher sich innerhalb von 20°C bis 70°C erstreckt, **dadurch gekennzeichnet, dass** mindestens eine der folgenden Temperaturen in dem Betriebstemperaturbereich liegt:
• Temperatur des wässrigen Mediums;
• Temperatur des Zwischenprodukts;
• Temperatur des Gemisches;
• Temperatur der getrennt erhaltenen ersten Phase.

9. Verfahren nach Anspruch 8, **dadurch kennzeichnet, dass** die Temperatur des Produkts unmittelbar nach dessen Erhalt in dem Betriebstemperaturbereich liegt.

10. Verfahren nach einem der Ansprüche 7 bis 9, wobei das Überführen des Zwischenproduktes in das Gemisch zumindest durch Abkühlen erfolgt, **dadurch kennzeichnet, dass** das Verfahren zusätzlich den folgenden Schritt umfasst:
g) Erwärmen der getrennt erhaltenen ersten Phase;
sodass die Temperatur des Gemisches niedriger ist als die Temperatur des Zwischenprodukts und dass die Temperatur der getrennt erhaltenen ersten Phase höher ist als die Temperatur des Gemisches.

11. Verfahren nach einem der vorhergehenden Ansprüche, **dadurch gekennzeichnet, dass** das Verfahren zusätzlich den folgenden Schritt umfasst:
h) Ausschleusen der getrennt erhaltenen zweiten Phase.

12. Verfahren nach einem der vorhergehenden Ansprüche, **dadurch gekennzeichnet, dass** innerhalb der elektrochemischen Zelle Wasser elektrochemisch gespalten wird, dass die dabei entstehenden Hydroxidionen OH⁻ in dem anodischen Kompartiment angereichert werden, und dass die dabei entstehenden Protonen H⁺ in dem kathodischen Kompartiment angereichert werden.

13. Verfahren nach einer der vorhergehenden Ansprüche, **dadurch gekennzeichnet, dass** das wässrige Medium durch Aufarbeitung einer Fermentationsbrühe bereitgestellt wird, wobei die Fermentationsbrühe zumindest vor ihrer Aufarbeitung mindestens einen Mikroorganismus enthält, wobei besagter Mikroorganismus ausgewählt ist aus der Gruppe bestehend aus den folgenden Mikroorganismen: *Actinobacteria, Bacillota, Pseudomonadota,* und/oder wobei es sich bei besagtem Mikroorganismus um *Pseudomonas aeruginosa* oder um *Pseudomonas alloputida* oder um *Pseudomonas putida* handelt.

14. Verfahren nach Anspruch 13, wobei im Rahmen der Aufarbeitung der Fermentationsbrühe ein organisches Hilfsmedium eingesetzt und wieder entfernt wird, sodass der Gehalt des organischen Hilfsmediums im wässrigen Medium kleiner ist als 5 Gew.-%, bezogen auf das Gewicht des wässrigen Mediums, wobei es sich bei dem organischen Hilfsmedium eine Substanz handelt, die aus der Gruppe bestehend der folgenden Substanzen ausgewählt ist: Methylacetat, Ethylacetat, Propylacetat, Isopropylacetat, Butylacetat, Isoamylacetat, Butan-1-ol, Butan-2-ol, Diethylether; oder wobei es sich bei dem organischen Hilfsmedium um ein Substanzgemisch umfassend mindestens zwei Substanzen der vorerwähnten Gruppe handelt, unter der Maßgabe, dass die Summe der Einzelgehalte aller in dem Substanzgemisch enthaltenen Substanzen kleiner ist als 5 Gew.-%, bezogen auf das Gewicht des wässrigen Mediums.

15. Verfahren nach Anspruch 14, **dadurch gekennzeichnet, dass** außerhalb der Aufarbeitung der Fermentationsbrühe keine der in besagter Gruppe aufgezählten Substanzen eingesetzt wird.
